# FASCICULE DE BREVET EUROPEEN

(11) **EP 1 429 808 B1**
(45) Date de publication et mention de la délivrance du brevet: **31.03.2010**
(21) Numéro de dépôt: 02791476.1
(22) Date de dépôt: 26.07.2002
(51) Int. Cl.: A61K 47/38, A61K 35/74

(54) **COMPOSITION SOLIDE CONTENANT DES SPORES DE BACTERIES NON PATHOGENES DU GENRE BACILLUS**
FESTE ZUSAMMENSETZUNG MIT NICHT-PATHOGENEN BAKTERIENSPOREN VOM BAZILLUSTYP
SOLID COMPOSITION CONTAINING BACILLUS-TYPE NON-PATHOGENIC BACTERIAL SPORES

(30) Priorité: 27.07.2001 IT MI20011632
(43) Date de publication de la demande: 23.06.2004
(73) Titulaire: Sanofi-Synthelabo Otc S.P.A., I-20122 Milano (IT)
(72) Inventeur: PRATO, Tiziano, I-21100 Varese (IT)
(74) Mandataire: Monain, Patrice
(86) Numéro de dépôt international: PCT/EP2002/008384
(87) Numéro de publication internationale: WO 2003/011341

(56) Documents cités:
- WO-A-99/49877
- GB-A- 1 061 894
- US-A- 4 935 353

## Description

La présente invention concerne une composition solide contenant des spores de bactéries non pathogènes, pour utilisation dans les domaines pharmaceutique, vétérinaire ou de la nutrition, notamment une composition contenant des spores de bactéries du genre Bacillus.

La formulation des spores aux fins de leur administration chez l'homme ou chez l'animal est difficile et problématique dès lors que, s'agissant de matériel vivant, si l'on adopte des procédés non appropriés on court le risque de perdre une partie considérable de principe actif.

Différentes compositions solides à base de spores sont connues. Elles sont réalisées selon différentes techniques, telles que, par exemple, la lyophilisation ou le "spray drying", de même que sont connues des suspensions liquides de spores ou de simples mélanges de spores avec des excipients conventionnels de la technique pharmaceutique, telles que par exemple WO 99/49877, où sont décrites des compositions de spores de bacilles produisant de l'acide lactique pour la réduction du cholestérol.

L'un des inconvénients principaux de l'art antérieur est la faible stabilité des compositions qui en résultent, facteur critique qui ne permet pas le stockage pendant des périodes raisonnablement longues et qui rend nécessaire le stockage et la conservation de la composition avant son emploi à de basses températures afin de garder autant que possible inaltérées les propriétés des spores.

Un autre inconvénient des compositions de l'art antérieur est la faible concentration de principe actif, de spores donc, qui peut être introduite dans la composition même.

Des compositions liquides à base de spores sont disponibles sur le marché, par exemple la suspension de spores de bacilles commercialisée en Italie depuis longtemps sous la marque «Enterogermina^{®}». Pour autant qu'elle soit efficace et appréciée depuis longtemps par les consommateurs, cette suspension ne peut pas contenir une concentration très élevée de principe actif (supérieure à 2 milliards de spores/5 ml).

Le but de la présente invention est de fournir une composition solide à base de spores de bactéries non pathogènes, ci-après dénommées simplement « spores », qui soit simple à produire, facile à stocker et qui contienne une quantité élevée de principe actif et stable dans le temps.

Il a été maintenant trouvé qu'en faisant adsorber les spores sur une matrice appropriée, en utilisant la technique du lit d'air fluidisé, on obtient une forme solide de spores à très haute concentration, facile à traiter au plan industriel et très stable.

Notamment, il a été trouvé que cette forme solide, ci-après dénommée « composition », permet de fixer à la matrice une quantité importante de spores en créant une composition solide à haute concentration dont la granulométrie et la surface spécifique la rendent particulièrement adaptée à la libération in vivo des spores tout au long du tractus gastro-intestinal.

De plus, il a été trouvé que la composition est dotée d'excellentes propriétés de fluidité et, de ce fait, elle est susceptible d'être traitée industriellement aux fins de sa formulation en compositions monodose ou multidose, sans qu'il soit nécessaire de la soumettre à des traitements ultérieurs.

Ainsi, selon un de ses aspects l'invention concerne une composition de spores de bactéries non pathogènes du genre Bacillus adsorbées sur une matrice formée d'au moins un composé insoluble dans l'eau et d'un dérivé de la cellulose, susceptible être obtenue par la technique du lit d'air fluidisé.

Plus particulièrement, l'invention concerne une composition de spores de bactéries non pathogènes du genre Bacillus susceptible d'être obtenue par un procédé qui comprend traiter selon la technique du lit d'air fluidisé une suspension liquide desdites spores avec une matrice formée d'un composé adsorbant insoluble dans l'eau et d'un dérivé de la cellulose.

Selon la présente invention, l'expression « composé adsorbant » désigne un composé chimique quelconque, ou un mélange de composés chimiques, qui puisse être ingéré et soit doté de propriétés adsorbantes ; de préférence, le composé adsorbant insoluble dans l'eau est choisi dans le groupe formé d'argiles, kaolin, carbonate de calcium, silices colloïdaux, silicate de magnésium et aluminium, le kaolin étant particulièrement avantageux.

D'après l'invention, l'expression « dérivé de la cellulose » désigne un dérivé quelconque de la cellulose qui puisse être ingéré, telles que, par exemple, la cellulose microcristalline, la méthylcellulose, l'hydroxypropylméthyl-cellulose, etc. dont de larges gammes commerciales sont disponibles, la cellulose microcristalline étant particulièrement préférée.

Il est entendu, même où cela n'est pas expressément indiqué, que tous les composants de la composition de l'invention sont du type pouvant être ingéré par l'homme et/ou l'animal, c'est à dire "non toxique". Notamment, la nature et la qualité des composants seront choisies en fonction de l'utilisation ultime de la composition et leurs genre et pureté seront donc appropriés et compatibles avec les emplois finals de la composition ; en conséquence, à titre illustratif, lorsque la composition est destinée au traitement pharmaceutique, le genre et la pureté des composants devront être acceptables au niveau pharmaceutique et ainsi de suite.

Par les expressions "spores adsorbées", "adsorption" ou "adsorber" on entend, selon la présente invention, la rétention des spores à la surface de la matrice d'où ledites spores sont libérables dans le tractus digéstif.

Les spores peuvent être rétenues sur la matrice par n'importe quel rattachement, à savoir par une liason possible quelconque (chimique, biologique, physique,etc.) selon le type de matrice utilisée.

Les quantités relatives des deux composants qui constituent la matrice peuvent varier dans une large fourchette. Par exemple, le rapport en poids entre le composé adsorbant et le dérivé de la cellulose peut être compris entre 90:10 et 10 :90, de préférence entre 70:30 et 30:70, encore plus préférablement entre 60:40 et 40:60, les deux composants étant avantageusement présents dans la matrice dans un rapport en poids d'environ 50 :50.

En tout état de cause, la matrice de l'invention est un mélange insoluble dans l'eau, inerte vers les spores, où par « inerte vers les spores » on entend qu'elle n'interfère pas négativement avec les spores.

Les spores pouvant être employées dans la présente invention sont de préférence des spores de bactéries non pathogènes particulièrement utiles dans les domaines pharmaceutique, vétérinaire et/ou de la nutrition. La composition de la présente invention peut contenir des spores d'un seul Bacillus ou des spores de différents Bacilles mélangés.

Selon un aspect préféré, la composition de l'invention comprend des spores de Bacillus subtilis ou de Bacillus clausii.

De préférence, la composition de l'invention comprend les spores d'une ou plusieurs souches de Bacillus clausii (dont la dénomination taxonomique antérieure était Bacillus subtilis), déposées en conformité au Traité de Budapest auprès du CNCM Institut Pasteur sous les numéros d'ordre : I-273, I-274, I-275, I-276.

Pour la préparation de la composition, on utilise la technique du lit d'air fluidisé bien connue à homme du métier.

Selon cette technique, on pulvérise une suspension aqueuse contenant les spores, ci-après dénommée « suspension concentrée», sur une matrice obtenue en mélangeant au moins un composé adsorbant et un dérivé de la cellulose, agitée constamment par un flux d'air pendant toute la durée du procédé, dans un outillage prévu à cet effet, tel que, par exemple, une machine à granuler au lit d'air fluidisé.

La suspension concentrée est aqueuse qui présente une très haute concentration en spores.

Selon un aspect particulièrement avantageux de l'invention, la suspension concentrée est obtenue par inoculation d'une ou plusieurs souches de Bacilles dans un milieu de culture (par exemple à base de peptones et sels mineraux), en incubant le mélange à une température appropriée pendant 48-72 heures en conditions aérobies, en séparant par centrifugation les cellules du milieu épuisé avec de l'eau distillée et ensuite en pasteurisant la suspension obtenue. Un exemple de telle préparation est donné dans la partie expérimentale.

La suspension concentrée ainsi préparée a une concentration de spores de Bacillus supérieure à 10 milliards par gramme, normalement comprise entre environ 15 et environ 25 milliards par gramme ou même plus.

D'une façon générale, la suspension concentrée est préférablement conservée après avoir été congelée à cause de son instabilité élevée à la température ambiante. Dans ce cas particulier, la suspension concentrée sera décongelée juste avant son emploi dans le procédé de la présente invention.

Alternativement, la suspension concentrée peut être une suspension extemporanée de spores conservées sous forme lyophilisée dans l'eau.

La température du flux d'air utilisé dans le procédé de la présente invention est comprise entre la température ambiante et la température maximale supportée par les spores ; selon un aspect préféré, le procédé a lieu de préférence à une température comprise entre 40° et 90°C, avantageusement entre 60 et 80°C.

Une fois achevée la pulvérisation et l'adsorption de la matrice concentrée, la composition obtenue est ultérieurement gardée en suspension au moyen du flux d'air chauffé jusqu'à ce qu'il ait atteint l'humidité résiduelle souhaitée, de préférence, jusqu'à ce que l'humidité soit inférieure à 3%, avantageusement inférieure ou égale à 2%.

La durée du processus est définie par la quantité de suspension concentrée à pulvériser, par la vitesse de pulvérisation ainsi que par la température du flux d'air. Normalement, pour le traitement de quantités autour de 30 kg de matrice, deux heures environ sont nécessaires pour achever le procédé.

Le procédé de préparation de la composition constitue un objet ultérieur de la présente invention.

Ainsi que cela est indiqué ci-dessus, la composition de l'invention peut être obtenue sous une forme hautement concentrée et présenter une concentration comprise, par exemple, entre 3 et 30 milliards de spores par gramme de composition finale, par exemple entre 5 et 20 milliards de spores par gramme de composition finale, avantageusement d'environ 10 milliards de spores par gramme, permettant ainsi l'administration de quantités consistantes de spores dans des petits volumes. Cette importante propriété fait que la composition est particulièrement facile à utiliser et, par exemple, peut être introduite dans de petites gélules ou sachets ou incorporée dans des aliments ou dans d'autres compositions. Au besoin, la composition peut également être administrée après avoir été mise à nouveau en suspension dans de l'eau ou dans d'autres liquides appropriés.

La composition de l'invention s'est avérée stable tout en gardant inaltéré son titre pendant longtemps, même à des températures supérieures à la température ambiante (environ 40°C).

La détermination du titre en spores de la composition de l'invention peut être menée selon un mode opératoire quelconque, par exemple par comptage sur des plaques de culture conventionnelles.

Comme indiqué ci-dessus, la composition de l'invention présente une large surface spécifique, grâce à une taille des particules très fine, jusqu'à atteindre 90 % des particules de la composition dont la taille des particules est inférieure ou égale à 130 micromètres, de préférence avec 60% des particules de la composition dont la taille des particules est inférieure ou égale à 60 micromètres.

De plus, la composition n'a pas d'odeur ni de saveur et peut être ainsi ajoutée à des aliments, boissons, ou à d'autres compositions sans altérer leur saveur originale.

Si l'on veut, la composition peut contenir des additifs opportunément choisis en fonction du consommateur final, de la modalité d'absorption ou du genre de traitement ultérieur auquel on souhaite la soumettre, à l'unique condition que les additifs soient inertes versus les spores.

Par exemple, il sera possible d'ajouter des agents lubrifiants, diluants, etc. ou tout autre agent susceptible d'en accroître l'écoulement ou d'exalter d'autres propriétés physiques particulières dans le but de faciliter les traitements ultérieurs de la composition.

Par exemple, au cas où on souhaiterait introduire la composition dans des capsules de gélatine dure, il pourrait être utile d'ajouter du stéarate de magnésium et/ou de la cellulose microcristalline.

Alternativement ou conjointement aux additifs précités, pourront être ajoutés des aromatisants susceptibles de conférer à la composition des parfums ou des saveurs particuliers.

Les composants ultérieurs éventuels peuvent être ajoutés à la matrice avant l'adsorption des spores ou tout simplement à la composition finale obtenue par le procédé de l'invention.

Au besoin, la composition peut faire l'objet de modifications ultérieures ; la composition peut donc être granulée selon les techniques bien connues au cas où on souhaiterait procéder à sa compression, ou être traitée de façon à obtenir des compositions à libération contrôlée selon les techniques bien connues, afin de modifier le temps de son relargage dans l'intestin.

La composition peut être administrée en quantités variables selon les besoins pour lesquels elle est administrée. D'une façon générale, en cas d'administration chez l'homme, on peut prévoir 10 milliards de spores/jour et même davantage, avantageusement de 1 à 8 milliards par jour, par exemple 2, 4 ou 6 milliards de spores par jour, l'administration étant possible en une seule prise ou de façon réitérée.

Aux fins de son administration, la composition de l'invention peut, le cas échéant, être formulée en unités de dosage ; par exemple, grâce à ses qualités, elle peut être facilement formulée en capsules de gélatine, telles que les gélules dans le format 0, 1 ou 2, choisies par l'expert de la branche selon le dosage.

Les unités de dosage, sous forme de gélules ou de sachets, contenant la composition de l'invention représentent un objet ultérieur de la présente invention.

Par exemple, ces unités de dosage peuvent contenir de 1 à 10 milliards desdites spores, avantageusement de 2 à 5 milliards, de 50 à 500 mg, par exemple de 50 à 250 mg de kaolin et par exemple de 50 à 600 mg, par exemple de 50 à 300 mg de cellulose microcristalline.

Ces unités de dosage peuvent être administrées une ou plusieurs fois par jour, selon la nécessité et la concentration de l'unité de dosage.

Par exemple, on peut préparer des unité de dosage contenant 5-7 milliards de spores, avantageusement environ 6 milliards de spores, et administrer ladite unité de dosage une seule fois par jour.

S'agissant de matériel vivant, il est évident que le titre microbiologique peut subir des variations; par conséquant un excès de spores de 10-20% par rapport à la dose prévue est préférablement rajouté dans la préparation de la composition. Compte-tenu de l'absence de toxicité du produit, un tel excès ne cause toutefois aucun problème.

Certains lots de la compositions de l'invention conditionnés dans le verre ou dans le polyéthylène ont été soumis à des études de stabilité pour en évaluer le comportement à des températures différentes (de 5°C à 40°C) et dégrés d'humidité (jusqu'à 75% d'humidité relative). Les résultats après 24 mois ont montré que le titre de la composition n'a pas été altérée d'une façon significative dans aucune des conditions expérimentées. En outre on a évalué aux mêmes conditions la résistance aux antibiotiques et les caractéristiques biochimiques qui se sont montrées conformes aux propriétés originales du produit.

La composition selon l'invention est utile dans les domaines pharmaceutique, vétérinaire et/ou de la nutrition.

Elle possède notamment les mêmes applications que le produit Enterogermina^{®} dans le commerce, en particulier la composition de l'invention exerce une action bénéfique sur l'intestin et sur le système immunitaire et est particulièrement adaptée pour le traitement et la prévention du dysmicrobisme intestinal et la dysvitaminose endogène ainsi qu'en traitement coadjuvant dans la récupération de la flore microbienne intestinale altérée suite à la thérapie antibiotique et à la chimiothérapie, ainsi qu'elle est adaptée par exemple pour son utilisation en association avec des antibiotiques pour combattre l'*Hélicobacter pylori*.

L'invention a également par objet un médicament contenant la composition de spores de bactéries telle que définie ci-dessus.

Les exemples indiqués ci-après illustrent l'invention sans pour autant la limiter.

### PREPARATION DE LA SUSPENSION CONCENTREE

On conduit une pré-fermentation de 600 ml d'une suspension de quatre souches de Bacillus clausii I-273, I-274, I-275 et I-276 (en égales proportions) à la concentration de 500 millions de spores/ml dans 30 litres de milieu de fermentation pendant 7 heures. On inocule la suspension pre-fermentée dans 1000 litres de milieu de fermentation à base de peptones et sels mineraux; on incube à 37°C pendant 48-72 heures en conditions aérobies, on sépare par centrifugation les cellules du milieu de culture avec de l'eau distillée jusqu'à obtenir un volume final de 100 litres et ensuite on pasteurise la suspension à 70°C pendant 30 minutes. On obtient ainsi une suspension concentrée contenant une concentration de spores de Bacillus clausii d'environ 20 milliards par gramme.

### EXEMPLE 1

Dans un système pour le procédé à lit d'air fluidisé, on charge 15 kg de kaolin de qualité pharmaceutique et 15 kg de cellulose microcristalline de qualité pharmaceutique et on chauffe le mélange pendant quelques minutes au moyen d'un flux d'air à 60 ° C. Sous une hotte au flux laminaire, on charge dans un contenant doté d'une pompe péristaltique reliée à des buses de nébulisation de 1,2 mm de diamètre et reliées à l'installation à lit d'air fluidisé, 15 kg d'une suspension aqueuse d'un mélange de spores de Bacillus clausii I-273, I-274, I-275 et I-276 (suspension concentrée préparée comme ci-dessus) à la concentration de 20 milliards de spores par gramme. On pulvérise donc la suspension concentrée de spores sur le mélange chauffé avec une pression de 2 bar et un débit de 135 ml/minute en gardant en suspension le mélange avec de l'air à 60°C. Environ 110 minutes après on arrête le système, on laisse refroidir et on récupère la composition. On obtient ainsi une composition présentant les caractéristiques suivantes :
- titre - déterminé par comptage sur plaque: 10 milliards de spores/g ;
- humidité résiduelle ≤ 2 % ;
- granulométrie - déterminée par un granulomètre laser Malvern^{®} dans de l'eau déminéralisée: 90% des particules < 100 µm
   60 % des particules < 50 µm
- surface spécifique: 3-5 m²/g.

### EXEMPLE 2

En opérant comme dcrit dans l'Exemple 1, mais en utilisant seulement la souche I-274 de Bacillus clausii, on obtient un produit ayant les caractéristiques suivantes:
- titre - déterminé par comptage sur plaque : 10 milliards de spores/g ;
- humidité résiduelle ≤ 2 % ;
- granulométrie - déterminée par un granulomètre laser Malvern^{®} dans de l'eau déminéralisée: 90% des particules < 130 µm
   60 % des particules < 60 µm
- surface spécifique: 3,5-5 m²/g.

### EXEMPLE 3

En opérant comme décrit dans l'Exemple 1, mais en utilisant seulement la souche I-276 de Bacillus clausii, on obtient un produit ayant les caractéristiques suivantes:
- titre de 12 milliards de spores/g ;
- humidité résiduelle ≤ 3 % ;
- granulométrie - déterminée par un granulomètre laser Malvern^{®} dans de l'eau déminéralisée: 90% des particules < 130 µm
   60 % des particules < 60 µm
- surface spécifique: 3-5 m²/g.

### EXEMPLE 4

A 240 g d'une composition de l'Exemple 1 on ajoute 57 g de cellulose microcristalline et 3 g de stéarate de magnésium. Après avoir été mélangée, la composition ainsi obtenue est distribuée dans des capsules operculées de gélatine dure de format 1, contenant chacune 300 mg de la composition suivante :
Matrice (Kaolin + Cellulose microcristalline)
contenant environ 2 milliards de spores de

| | | |
|---|---|---|
| Bacillus Clausii (I-273, I-274, I-275, I-276) | mg | 240,00 |
| Cellulose microcristalline | mg | 57,00 |
| Stéarate de magnésium | mg | 3,00 |

### EXEMPLE 5

### CAPSULES DE GELATINE DURE

### FORMAT 1 CONTENANT 275 mg DE COMPOSITION

| | | |
|---|---|---|
| Matrice (Kaolin + Cellulose microcristalline) contenant environ 2 milliards de spores de Bacillus Clausü (I-273, I-274, I-275, I-276) | mg | 200,00* |
| Cellulose microcristalline | mg | 72,25 |
| Stéarate de magnésium d'origine végétale | mg | 2,75 |

| | | |
|---|---|---|
| (* dans la formule de fabrication 220,00 mg correspondant à un surdosage de 10%) | | |

### EXEMPLE 6

### CAPSULES DE GELATINE DURE

### FORMAT 1 CONTENANT 255 mg DE COMPOSITION

| | | |
|---|---|---|
| Matrice (Carbonate de calcium + Cellulose microcristalline) contenant environ 2 milliards de spores de Bacillus Clausü (I-273, I-274, I-275, I-276) | mg | 200,00* |
| Cellulose microcristalline | mg | 52,25 |
| Stéarate de magnésium d'origine végétale | mg | 2,75 |

| | | |
|---|---|---|
| (* dans la formule de fabrication 220,00 mg correspondant à un surdosage de 10%) | | |

## Revendications

1. Composition solide de spores de bactéries non pathogènes du genre Bacillus adsorbées sur une matrice formée :
- d'au moins un composé adsorbant insoluble dans l'eau choisi dans le groupe formé d'argiles, kaolin, carbonate de calcium, silices colloïdaux et silicate de magnésium et aluminium, et
- d'un dérivé de la cellulose,
**caractérisée en ce que** :
le rapport en poids entre ledit composé adsorbant et ledit dérivé de la cellulose est compris entre 90 :10 et 10 : 90, et
la composition est susceptible d'être obtenue par la technique du lit d'air fluidisé.

2. Composition selon la revendication 1, **caractérisée en ce qu'**elle est susceptible d'être obtenue par un procédé qui comprend traiter, selon la technique du lit d'air fluidisé, une suspension liquide desdites spores avec une matrice formée d'au moins un composé adsorbant insoluble dans l'eau et d'un dérivé de la cellulose.

3. Composition selon les revendications 1 ou 2, **caractérisée en ce qu'**elle contient de 3 à 30 milliards desdites spores par gramme de composition.

4. Composition selon la revendication 3, **caractérisée en ce qu'**elle contient de 5 à 20 milliards desdites spores par gramme de composition.

5. Composition selon la revendication 4, **caractérisée en ce qu'**elle contient environ 10 milliards desdites spores par gramme de composition.

6. Composition selon l'une des revendications de 1 à 5, **caractérisée en ce que** ledit composé adsorbant est le kaolin.

7. Composition selon l'une des revendications de 1 à 5, **caractérisée en ce que** ledit composé adsorbant est le carbonate de calcium.

8. Composition selon l'une des revendications de 1 à 7, **caractérisée en ce que** ledit dérivé de la cellulose est la cellulose microcristalline.

9. Composition selon l'une des revendications de 1 à 8, **caractérisée en ce que** le rapport en poids entre ledit composé adsorbant et ledit dérivé de cellulose est compris entre 70 :30 et 30 :70.

10. Composition selon la revendication 9 **caractérisée en ce que** ledit rapport est environ 50 :50.

11. Composition selon l'une des revendications de 1 à 10 sous forme d'unité de dosage en gélules ou sachets.

12. Composition selon la revendication 11, contenant pour chaque unité de dosage de 1 à 10 milliards desdites spores, de 50 à 500 mg de kaolin et de 50 à 600 mg de cellulose microcristalline.

13. Composition selon la revendication 12, contenant pour chaque unité de dosage de 2 à 5 milliards desdites spores, de 50 à 250 mg de kaolin et de 50 à 300 mg de cellulose microcristalline.

14. Composition selon la revendication 11, contenant pour chaque unité de dosage environ 2 milliards desdites spores en gélules.

15. Composition selon la revendication 11, contenant pour chaque unité de dosage environ 6 milliards desdites spores en gélules.

16. Composition selon l'une des revendications 1 à 15, **caractérisée en ce que** lesdites spores sont des spores de Bacillus subtilis ou de Bacillus clausii.

17. Composition selon la revendication 16, **caractérisée en ce que** lesdites spores proviennent d'un ou plusieurs souches choisies parmi Bacillus clausii I-273, I-274, I-275 et I-276.

18. Composition selon l'une des revendications 1 à 15, **caractérisée en ce que** lesdites spores sont un mélange de spores de Bacilles différents.

19. Médicament comprenant la composition selon l'une des revendications de 1 à 18.

## Claims

1. Solid composition of spores of nonpathogenic bacteria of the Bacillus genus, adsorbed onto a matrix made up of:
- at least one water-insoluble adsorbent compound chosen from the group made up of clays, kaolin, calcium carbonate, colloidal silicas and magnesium and aluminium silicate, and
- a cellulose derivative,
**characterized in that**:
the ratio by weight of said adsorbent compound to said cellulose derivative is between 90:10 and 10:90, and
the composition can be obtained by the fluidized air bed technique.

2. Composition according to Claim 1, **characterized in that** it can be obtained by a process which comprises treating, according to the fluidized air bed technique, a liquid suspension of said spores with a matrix made up of at least one water-insoluble adsorbent compound and a cellulose derivative.

3. Composition according to Claim 1 or 2,
**characterized in that** it contains from 3 to 30 billion of said spores per gram of composition.

4. Composition according to Claim 3, **characterized in that** it contains from 5 to 20 billion of said spores per gram of composition.

5. Composition according to Claim 4, **characterized in that** it contains approximately 10 billion of said spores per gram of composition.

6. Composition according to one of Claims 1 to 5, **characterized in that** said adsorbent compound is kaolin.

7. Composition according to one of Claims 1 to 5, **characterized in that** said adsorbent compound is calcium carbonate.

8. Composition according to one of Claims 1 to 7, **characterized in that** said cellulose derivative is microcrystalline cellulose.

9. Composition according to one of Claims 1 to 8, **characterized in that** the ratio by weight of said adsorbent compound to said cellulose derivative is between 70:30 and 30:70.

10. Composition according to Claim 9, **characterized in that** said ratio is approximately 50:50.

11. Composition according to one of Claims 1 to 10, in the form of a dosage unit in gel capsules or sachets.

12. Composition according to Claim 11, containing for each dosage unit from 1 to 10 billion of said spores, from 50 to 500 mg of kaolin and from 50 to 600 mg of microcrystalline cellulose.

13. Composition according to Claim 12, containing for each dosage unit from 2 to 5 billion of said spores, from 50 to 250 mg of kaolin and from 50 to 300 mg of microcrystalline cellulose.

14. Composition according to Claim 11, containing for each dosage unit approximately 2 billion of said spores in gel capsules.

15. Composition according to Claim 11, containing for each dosage unit approximately 6 billion of said spores in gel capsules.

16. Composition according to one of Claims 1 to 15, **characterized in that** said spores are spores of Bacillus subtilis or of Bacillus clausii.

17. Composition according to Claim 16, **characterized in that** said spores originate from one or more strains chosen from Bacillus clausii I-273, I-274, I-275 and I-276.

18. Composition according to one of Claims 1 to 15, **characterized in that** said spores are a mixture of spores of different Bacilli.

19. Medicinal product comprising the composition according to one of Claims 1 to 18.

## Patentansprüche

1. Feste Zusammensetzung mit nichtpathogenen Bakteriensporen des Bacillus-Typs, die an eine aus:
- mindestens einer wasserunlöslichen adsorbierenden Zusammensetzung aus der Gruppe Tonmineralien, Kaolin, Calciumcarbonat, kolloidale Kieselerden und Magnesium-Aluminium-Silikat und
- einem Cellulosederivat gebildete Matrix adsorbiert sind, **dadurch gekennzeichnet, dass**
das Gewichtsverhältnis zwischen der adsorbierenden Verbindung und dem Cellulosederivat zwischen 90:10 und 10:90 beträgt und
die Zusammensetzung mit der Luftwirbelbetttechnik erhältlich ist.

2. Zusammensetzung nach Anspruch 1, **dadurch gekennzeichnet, dass** sie mit einem Verfahren erhältlich ist, bei dem eine flüssige Suspension der genannten Sporen mit einer aus mindestens einer wasserunlöslichen adsorbierenden Verbindung und einem Cellulosederivat gebildeten Matrix mit der Luftwirbelbetttechnik behandelt wird.

3. Zusammensetzung nach Anspruch 1 oder 2, **dadurch gekennzeichnet, dass** sie 3 bis 30 Milliarden der Sporen pro Gramm Zusammensetzung enthält.

4. Zusammensetzung nach Anspruch 3, **dadurch gekennzeichnet, dass** sie 5 bis 20 Milliarden der Sporen pro Gramm Zusammensetzung enthält.

5. Zusammensetzung nach Anspruch 4, **dadurch gekennzeichnet, dass** sie ungefähr 10 Milliarden der Sporen pro Gramm Zusammensetzung enthält.

6. Zusammensetzung nach einem der Ansprüche 1 bis 5, **dadurch gekennzeichnet, dass** es sich bei der adsorbierenden Verbindung um Kaolin handelt.

7. Zusammensetzung nach einem der Ansprüche 1 bis 5, **dadurch gekennzeichnet, dass** es sich bei der adsorbierenden Verbindung um Calciumcarbonat handelt.

8. Zusammensetzung nach einem der Ansprüche 1 bis 7, **dadurch gekennzeichnet, dass** es sich bei dem Cellulosederivat um mikrokristalline Cellulose handelt.

9. Zusammensetzung nach einem der Ansprüche 1 bis 8, **dadurch gekennzeichnet, dass** das Gewichtsverhältnis zwischen der adsorbierenden Verbindung und dem Cellulosederivat zwischen 70:30 und 30:70 beträgt.

10. Zusammensetzung nach Anspruch 9, **dadurch gekennzeichnet, dass** das Verhältnis ungefähr 50:50 beträgt.

11. Zusammensetzung nach einem der Ansprüche 1 bis 10 in Einzeldosisform als Weichgelatinekapseln oder Briefchen.

12. Zusammensetzung nach Anspruch 11, die pro Einzeldosis von 1 bis 10 Milliarden der Sporen 50 bis 500 mg Kaolin und 50 bis 600 mg mikrokristalline Cellulose enthält.

13. Zusammensetzung nach Anspruch 12, die pro Einzeldosis von 2 bis 5 Milliarden der Sporen 50 bis 250 mg Kaolin und 50 bis 300 mg mikrokristalline Cellulose enthält.

14. Zusammensetzung nach Anspruch 11, die pro Einzeldosis ungefähr 2 Milliarden der Sporen in Weichgelatinekapseln enthält.

15. Zusammensetzung nach Anspruch 11, die pro Einzeldosis ungefähr 6 Milliarden der Sporen in Weichgelatinekapseln enthält.

16. Zusammensetzung nach einem der Ansprüche 1 bis 15, **dadurch gekennzeichnet, dass** es sich bei den Sporen um Sporen von Bacillus subtilis oder von Bacillus clausii handelt.

17. Zusammensetzung nach Anspruch 16, **dadurch gekennzeichnet, dass** die Sporen von einer oder mehreren Stämmen aus der Reihe Bacillus clausii 1-273, I-274, I-275 und I-276 stammen.

18. Zusammensetzung nach einem der Ansprüche 1 bis 15, **dadurch gekennzeichnet, dass** es sich bei den Sporen um eine Mischung von Sporen von verschiedenen Bacillen handelt.

19. Arzneimittel, das die Zusammensetzung nach einem der Ansprüche 1 bis 18 enthält.
